(19) Europäisches Patentamt

European Patent Office

Office européen des brevets

(11) Veröffentlichungsnummer: **0 290 923 B1**

(12) **EUROPÄISCHE PATENTSCHRIFT**

(45) Veröffentlichungstag der Patentschrift: **29.07.92**

(21) Anmeldenummer: **88107073.4**

(22) Anmeldetag: **03.05.88**

(51) Int. Cl.⁵: **C12M 1/00**, B01L 7/00

(54) **Klimaschrank.**

(30) Priorität: **12.05.87 CH 1814/87**

(43) Veröffentlichungstag der Anmeldung:
**17.11.88 Patentblatt 88/46**

(45) Bekanntmachung des Hinweises auf die
Patenterteilung:
**29.07.92 Patentblatt 92/31**

(84) Benannte Vertragsstaaten:
**DE ES FR GB IT SE**

(56) Entgegenhaltungen:
**EP-A- 0 019 828**
**US-A- 3 712 268**
**US-A- 4 039 775**

(73) Patentinhaber: **SALVIS AG**
**Hauptstrasse 49**
**CH-6015 Reussbühl(CH)**

(72) Erfinder: **Süess, Josef**
**Ruopigenring 107/38**
**CH-6015 Reussbühl(CH)**
Erfinder: **Ghezzi, Renato**
**Fichtenstrasse 30**
**CH-5032 Emmen(CH)**

(74) Vertreter: **Blum, Rudolf Emil Ernst et al**
**c/o E. Blum & Co Patentanwälte Vorderberg**
**11**
**CH-8044 Zürich(CH)**

# Beschreibung

Die vorliegende Erfindung bezieht sich auf einen Klimaschrank mit einem einen Nutzraum bildenden Nutzraumgehäuse und einer dieses abschliessenden Türe.

Klimaschränke werden in den verschiedensten Bereichen, z.B. als Kühl-, Trocken-, Warme- oder Brutschränke eingesetzt.

Bei herkömmlichen Modellen besteht das Nutzraungehäuse aus einem metallenen Innengehäuse (meist Aluminium- oder CNS-Schweiss-/Kantkonstruktionen), an dem z.B. Heizelemente zur Beheizung des Behandlungsraums angebracht sind. Zur Vermeidung von Energieverlusten wird das Innengehäuse mit einer Isoliermatte umgeben und von einem metallenen Aussengehäuse (lackiert oder CNS) umschlossen. Die erforderlichen Funktions- und Bedienungselemente sind oben, seitlich oder unten an diesem Gehäuse untergebracht. Das Nutzraumgehäuse ist frontseitig durch eine aus einer isolierten Metallhülle bestehende Türe, welche als Bedienungsöffnung dient, abgeschlossen. Bei dieser Konstruktionsweise lassen sich Wärmeverluste und Geruchsimmissionen aufgrund von undichten Stellen nur mittels aufwendiger und kostspieliger Abdichtungen vermeiden. Direkte Metallkontakte oder ungenügende Abstände zwischen Innen- und Aussengehäuse führen konstruktionsbedingt zu Warmbrücken. Die thermische Trägheit des metallenen Innengehäuses hat beträchtliche Energieinvestitionen zur Folge. Ein solcher Klimaschrank ist schwer und muss aus einer Vielzahl von Einzelteilen gefertigt werden, was sich wiederum in den Kosten niederschlägt. Wegen der rechtwinkligen Innenkanten ist die Reinigung schwierig, in bestimmten Anwendungsfällen (z.B. Bakterienzucht) sogar unmöglich. Für das Bedienungspersonal besteht zudem eine gewisse Verbrennungsgefahr beim Berühren des heissen Innengehäuses. Wartungsarbeiten sind dadurch erschwert, dass die im Nutzraumgehäuse untergebrachten Funktions- und Bedienungselemente meist nur schwer zugänglich sind.

Es stellt sich damit die Aufgabe, einen Klimaschrank zu schaffen, der sowohl absolut dicht und optimal isolierend ist und der zudem eine hohe Bedienungs-, Reinigungs- und Wartungsfreundlichkeit garantiert und gleichzeitig unter den heute üblichen Herstellkosten produziert werden kann.

Zu diesem Zweck schlägt die Erfindung einen Klimaschrank vor, bei dem die Funktionselemente für die Erzeugung, Steuerung und Ueberwachung des Nutzraumklimas sowie die entsprechenden Bedienungselemente in der Türe angeordnet sind.

Dadurch erübrigen sich bei dem den Behandlungsraum umgebenden Gehäuse Oeffnungen oder Aussparungen für die Aufnahme von Funktions- und Bedienungselementen, weshalb auf die herkömmliche Metallbauweise verzichtet und das Nutzraumgehäuse aus Kunststoff oder Keramik gefertigt werden kann.

Ein Vorteil dieser Erfindung ist darin zu sehen, dass das Gehause aus einem einzigen (bei grösseren Geräten einigen wenigen) Kunststoff- oder Keramikteil bestehen kann, wodurch bei gleichbleibendem Nutzvolumen und kleineren Aussenmassen bedeutend geringere Wärmeverluste bei gleichzeitiger Gasdichtheit erzielt werden. Bei gleichem Nutzvolumen sind im Vergleich zu herkömmlichen Geräten in der Breite wesentlich geringere Aussenmasse erzielbar. Damit kann Laborplatz eingespart werden, wobei die meist ausreichend zur Verfügung stehende Tiefe voll genutzt werden kann. Die thermische Trägheit sowie die Verbrennungsgefahr können stark reduziert werden. Sämtliche Innenkanten können gerundet sein, was zu hoher Reinigungsfreundlichkeit führt. Die Anzahl der Baugruppen wird stark reduziert, die Montage vereinfacht, wodurch die Herstellkosten eine massive Senkung erfahren. Wegen der Konzentration der Betriebselemente in der Türe sind nur kurze Verbindungsleitungen notwendig. Die Wartung wird zudem erheblich erleichtert.

Im folgenden soll eine mögliche Ausführung der Erfindung anhand von Zeichnungen erläutert werden. Dabei zeigt:

Figur 1 einen Horizontalschnitt durch einen Klimaschrank;

Figur 2 eine Frontansicht der Ture desselben Klimaschranks und

Figur 3 schematisch einen aus zwei Gehäuseteilen bestehenden Klimaschrank.

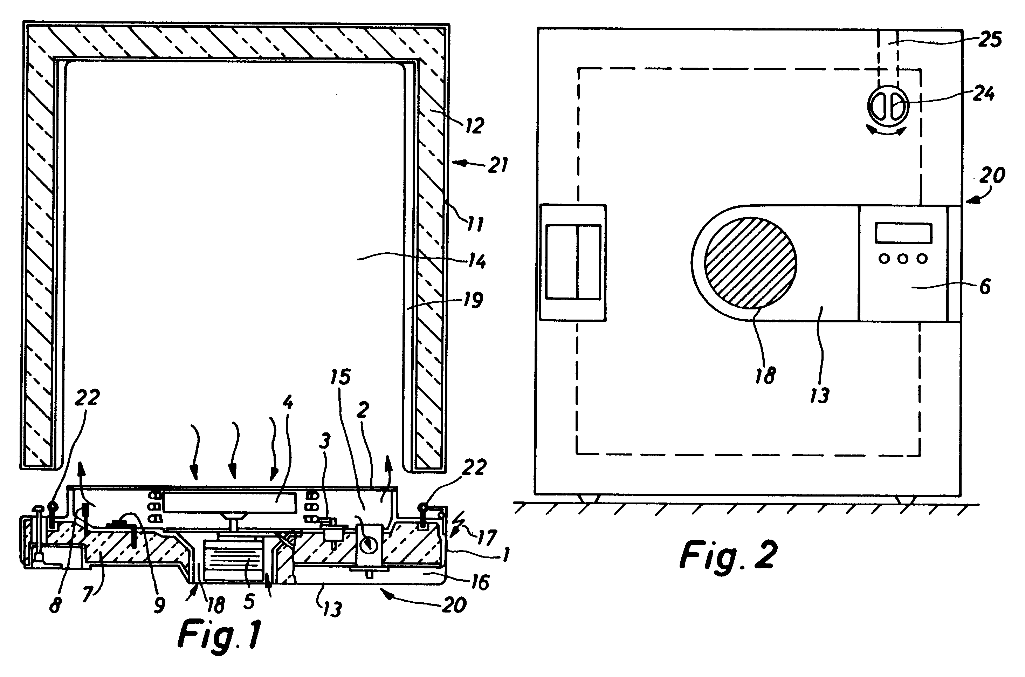

Die Figuren 1 und 2 zeigen einen erfindungsgemassen Klimaschrank, der speziell als Warmeschrank ausgelegt ist, mit einem Nutzraumgehause 21 und einer Türe 20, welche aus zeichnerischen Gründen losgelöst vom Nutzraumgehäuse 21 dargestellt ist. Sämtliche Funktionseinheiten sind in der Türe 20 untergebracht. Die Türe 20 besteht aus einem Tragrahmen 1, auf dessen innerer Seite sich eine Heiz- und Luftleitzone 15 befindet. Diese enthält ein Heizelement 3, einen Ventilator 4, einen Temperaturfühler 8 sowie eine Temperatursicherung 9 und wird durch eine Luftleitplatte 2 begrenzt. Kleinere Modelle (bis ca. 100 Liter Nutzvolumen) können auch ohne Ventilator 4 durch Ausnutzen der Thermik genügend genau betrieben werden.

Auf der Aussenseite des Tragrahmens 1 befindet sich eine Isolierkammer 16, welche mit einer Wärmeisolierschicht 7 versehen ist. Ueber der Isolierschicht 7 ist eine Frontblende 13 montiert, welche eine Regel- und Bedienungseinheit 6 trägt. Die Stromzufuhr erfolgt über ein in den Tragrahmen 1 montiertes Netzanschlusskabel 17. Die für Trocknungsprozesse erforderliche Frischluft wird über eine Motorenkammer 18, welche einen Ventilatormotor 5 enthält, zugeführt. Mittels einer Abluftregulierung 24 kann der Luftaustritt durch eine Abluftöffnung 25 bestimmt werden.

Das Nutzraumgehäuse 21 besteht aus einem doppelwandigen monolithischen Gehäuseteil 11, welcher einen Nutzraum 14 umschliesst und eine thermisch isolierende Schicht 12 enthält. Dieser Aufbau ermöglicht - in Zusammenwirkung mit Türabdichtungen 22 - den Nutzraum 14 völlig gasdicht abzuschliessen. Der Gehäuseteil 11 weist auf seiner Innenseite gerundete Tablartäger 19 auf, welche als Ausformungen der Innenhülle des Gehäuseteils 11 ausgebildet sind.

Der Tragrahmen 1 sowie der Gehäuseteil 11 bestehen aus Kunststoff (z.B. Polyphenylsulfid, das auch bei hohen Temperaturen eine einwandfreie Formstabilität aufweist) oder aus Keramik, die Isolierschicht 12 beispielsweise aus PIR-Schaum. Damit wird eine Temperaturbeständigkeit bis ca. 240° C erreicht, womit rund 95% der Anwendungen abgedeckt werden können.

Die Herstellung des Nutzraumgehäuses 21 beschränkt sich im wesentlichen auf bloss zwei Arbeitsgänge, indem in einem ersten Arbeitsgang mittels Spritzgussverfahren der Gehäuseteil 11 hergestellt und dieser anschliessend in einem zweiten Arbeitsgang mit dem Isoliermaterial 12 ausgeschäumt wird.

Bei grossen Modellen kann der Gehäuseteil 11 aus Transportgründen auch aus mehreren Teilen bestehen, welche erst nachträglich zusammengesetzt werden.

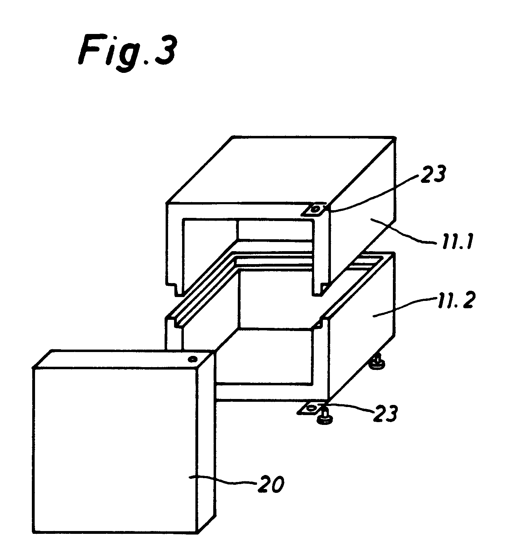

Figur 3 zeigt schematisch einen aus zwei Gehäuseteilen 11.1, 11.2 bestehenden Klimaschrank, bei welchem zudem die Anordnung der Tür-Scharniere 23 ersichtlich ist.

Gesamthaft zeichnet sich diese Bauweise durch eine hohe Wartungsfreundlichkeit aus, indem nach Entfernen der Frontblende 13 die in der Türe 20 integrierten Funktionselemente sofort zugänglich sind. Zudem kann durch einfaches Auswechseln der Türe die Einsatzbereitschaft eines defekten Gerätes sofort wieder hergestellt werden. Selbstverständlich sind auch Geräte mit erweiterten Betriebsfunktionen ohne weiteres denkbar, beispielsweise indem Kühlelemente oder Vorrichtungen für definiertes Fluten des Nutzraumes 14 mit Gasen (z.B. $N_2$) in die Türe 20 integriert werden.

**Patentansprüche**

1. Klimaschrank mit einem einen Nutzraum (14) bildenden Nutzraumgehäuse (21) und einer dieses abschliessenden Türe (20), dadurch gekennzeichnet, dass die Funktionselemente für die Erzeugung, Steuerung und Ueberwachung des Nutzraumklimas sowie die entsprechenden Bedienungselemente in der Türe (20) angeordnet sind.

2. Klimaschrank nach Anspruch 1, dadurch gekennzeichnet, dass die Türe (20) einen die Funktions- und Bedienungselemente tragenden Rahmen (1) aufweist.

3. Klimaschrank nach Anspruch 2, dadurch gekennzeichnet, dass dieser Rahmen (1) aus Kunststoff oder Keramik besteht.

4. Klimaschrank nach einem der vorangehenden Ansprüche, dadurch gekennzeichnet, dass die Türe (20) auf ihrer Aussenseite eine wegnehmbare Frontblende (13) aufweist, wodurch die Funktionselemente jederzeit zugänglich sind.

5. Klimaschrank nach einem der vorangehenden Ansprüche, dadurch gekennzeichnet, dass die Türe (20) eine Lufteinlassöffnung (18) für die Versorgung des Nutzraumes (14) mit Frischluft sowie eine regulierbare Luftaustrittsöffnung (25) aufweist.

6. Klimaschrank nach einem der vorangehenden Ansprüche mit einem Nutzraumgehäuse (21), dessen Wandungen durch eine Innen- und eine Aussenhülle mit dazwischenliegender Isolationsschicht (12) gebildet werden, dadurch gekennzeichnet, dass die Innen- und Aussenhülle aus Kunststoff oder Keramik bestehen.

7. Klimaschrank nach Anspruch 6, dadurch gekennzeichnet, dass die Innen- und die Aussenhülle des Nutzraumgehäuses (21) durch einen einzigen monolithischen Gehäuseteil (11) gebildet werden.

8. Klimaschrank nach Anspruch 6, dadurch gekennzeichnet, dass die Innen- und Aussenhülle durch einen oberen und einen unteren Gehäuseteil (11.1, 11.2) gebildet werden.

9. Klimaschrank nach einem der vorangehenden Ansprüche, dadurch gekennzeichnet, dass sämtliche Innenkanten und -ecken des Nutzraumgehäuses (21) gerundet sind.

10. Klimaschrank nach einem der Ansprüche 6 bis 9, dadurch gekennzeichnet, dass im Innern des Nutzraumgehäuses (21) gerundete Tablarträger (19) vorgesehen sind, welche durch Ausformungen der Innenhülle gebildet werden.

11. Klimaschrank nach einem der Ansprüche 3 oder 6, dadurch gekennzeichnet, dass der genannte Kunststoff für den Rahmen (1) der Türe (20) bzw. für die Innen- und Aussenhülle des Nutzraumgehäuses (21) Polyphenylsulfid ist.

12. Klimaschrank nach Anspruch 6, dadurch gekennzeichnet, dass die Isolationsschicht (12) aus PIR-Schaum besteht.

## Claims

1. Climate chamber with a housing (21) which delimits a working chamber (14) and a door (20) which closes the same, characterized in that the functional elements for producing, commanding and monitoring the climate in the working chamber, and also the corresponding controls are located in the door (20).

2. Climate chamber according to claim 1, characterized in that the door (20) comprises a frame (1) which supports the functional elements and the controls.

3. Climate chamber according to claim 2, characterized in that said frame (1) consists of ceramic or plastic.

4. Climate chamber according to any preceding claim, characterized in that the door (20) comprises on its outer side a removable frontal cover (13) so that the functional elements remain always accessible.

5. Climate chamber according to any preceding claim, characterized in that the door (20) comprises an air intake (18) for supplying fresh air into the working chamber (14), and also an exhaust aperture (25) which can be regulated.

6. Climate chamber according to any preceding claim with a housing (21), the walls of which are composed of an inner and an outer casing with an insulating layer (12) in between, characterized in that the inner and outer casing consist of plastic or of ceramic.

7. Climate chamber according to claim 6, characterized in that the inner and the outer casing of the housing (21) are formed by a single, monolithic housing element (11).

8. Climate chamber according to claim 6, characterized in that the inner and the outer casing are formed by an upper and a lower housing element (11.1, 11.2).

9. Climate chamber according to any preceding claim, characterized in that all inner edges and corners of the housing (21) are rounded.

10. Climate chamber according to any of claims 6 through 9, characterized in that rounded shelf supports (19) are provided inside the housing (21), these supports being obtained by shaping the inner casing.

11. Climate chamber according to one of claims 3 or 6, characterized in that said plastic for the frame (1) of the door (20), respectively for the inner and outer casing of the housing (21) is polyphenylsulfide.

12. Climate chamber according to claim 6, characterized in that the insulating layer (12) is made of PIR-foam.

## Revendications

1. Armoire climatisée avec un logement (21) formant une enceinte de travail (14) et une porte (20) pour clore celle-ci, caractérisée en ce que les éléments fonctionnels pour produire, régler et surveiller l'atmosphère dans l'enceinte de travail, ainsi que les organes de commande correspondants sont agencés dans la porte (20).

2. Armoire climatisée selon la revendication 1, caractérisée en ce que la porte (20) comporte un cadre (1) supportant les éléments fonctionnels et les organes de commande.

3. Armoire climatisée selon la revendication 2, caractérisée en ce que le cadre (1) est en matière plastique ou en céramique.

4. Armoire climatisée selon une des revendications précédentes, caractérisée en ce que la porte (20) comporte sur son côté extérieur un écran frontal (13) amovible, de manière à ce que les éléments fonctionnels soient toujours accessibles.

5. Armoire climatisée selon une des revendications précédentes, caractérisée en ce que la porte (20) comporte une entrée d'air (18) pour l'alimentation de l'enceinte (14) en air frais, ainsi qu'une sortie d'air (25) réglable.

6. Armoire climatisée selon une des revendications précédentes avec un logement (21) dont les parois sont composées d'une enveloppe extérieure et d'une enveloppe intérieure ainsi que d'une couche d'isolation (12) placée entre les deux, caractérisée en ce que les enveloppes extérieure et intérieure sont en céramique ou en matière plastique.

7.  Armoire climatisée selon la revendication 6, caractérisée en ce que les enveloppes extérieure et intérieure du logement (21) sont formées d'un seul élément monolithique (11).

8.  Armoire climatisée selon la revendication 6, caractérisée en ce que les enveloppes extérieure et intérieure sont formées par un élément de logement supérieur et un élément de logement inférieur (11.1, 11.2).

9.  Armoire climatisée selon une des revendications précédentes, caractérisée en ce que toutes les arêtes et tous les coins intérieurs du logement (21) sont arrondis.

10. Armoire climatisée selon une des revendications 6 à 9, caractérisée en ce que des porte-étagères (19) arrondis formés par des bossages de l'enveloppe intérieure sont prévus à l'intérieur du logement (21).

11. Armoire climatisée selon une des revendications 3 ou 6, caractérisée en ce que ladite matière plastique pour le cadre (1) de la porte (20), respectivement pour l'enveloppe intérieure et extérieure du logement (21) est du polyphénylenesulfide.

12. Armoire climatisée selon la revendication 6, caractérisée en ce que la couche isolante (12) est une mousse PIR.

Fig.1

Fig. 2

EP 0 290 923 B1

# Fig.3